# EUROPEAN PATENT SPECIFICATION

(11) **EP 4 480 629 B1**
(45) Date of publication and mention of the grant of the patent: **08.04.2026**
(21) Application number: 24181524.0
(22) Date of filing: 11.06.2024
(51) Int. Cl.: B23K 31/12, G01N 29/24

(54) **A WELDING INSPECTION METHOD OF INSPECTING A WELD**
VERFAHREN ZUR SCHWEISSPRÜFUNG ZUR PRÜFUNG EINER SCHWEISSWEISE
PROCÉDÉ D'INSPECTION DE SOUDURE POUR INSPECTER UNE SOUDURE

(30) Priority: 16.06.2023 JP 2023098925
(43) Date of publication of application: 25.12.2024
(73) Proprietor: DAIHEN Corporation, Yodogawa-ku Osaka-shi, Osaka 532-8512 (JP); The University of Osaka, Suita-shi, Osaka 565-0871 (JP)
(72) Inventor: Nitta, Seiya, Osaka (JP); Kadota, Keiji, Osaka (JP); Tanaka, Toshiyuki, Osaka (JP); Asai, Satoru, Osaka (JP); Nomura, Kazufumi, Osaka (JP)
(74) Representative: Zacco GmbH

(56) References cited:
- EP-A1- 4 124 859
- CN-A- 113 049 677
- US-A1- 2018 321 485

## Description

### BACKGROUND OF THE INVENTION

### Field of the Invention

The present disclosure relates to a welding inspection method of inspecting a weld, see claim 1.

### Description of the Background Art

For example, Japanese Patent No. 5651533 discloses a welding inspection method for non-destructive detection of an internal defect such as a blowhole produced in a weld. In this welding inspection method, the internal defect in the weld is detected by irradiation from above a weld bead, of a bead surface with transmission laser light and detection of ultrasound (scattered waves) reflected by the defect.

EP 4 124 859 Al describes also such method, e.g. detection of an internal defect in a weld seam by irradiation with laser light for excitation of ultrasound inside the weld.

### SUMMARY OF THE INVENTION

In the welding inspection method disclosed in Japanese Patent No. 5651533, there is a concern that presence of an impurity such as slag on an irradiation surface of a weld bead irradiated with transmission laser light interferes with generation of ultrasound and becomes a factor for defective inspection.

Therefore, an object of the present invention is to provide a welding inspection method that allows appropriate detection of an internal defect in a weld even when an impurity is attached to a surface of the weld.

According to a first aspect of the present invention, a welding inspection method of inspecting a weld is defined in claim 1.

According to this welding inspection method, the impurity attached to the surface of the weld is removed before irradiation of the surface of the weld with transmission laser light. Therefore, even when the impurity is attached to the surface of the weld, the internal defect in the weld can appropriately be detected.

Further preferred embodiments of the first aspect of the present invention are defined in the dependent claims.

The foregoing and other objects, features, aspects and advantages of this invention will become more apparent from the following detailed description of this invention when taken in conjunction with the accompanying drawings.

### BRIEF DESCRIPTION OF THE DRAWINGS

Fig. 1 is an overall configuration diagram (No. 1) of a welding system to which a welding inspection method is applied.
Fig. 2 is a diagram (No. 1) illustrating principles of detection of an internal defect in a weld.
Fig. 3 is a diagram (No. 2) illustrating principles of detection of the internal defect in the weld.
Fig. 4 is a diagram showing relation between a position of irradiation with transmission laser light and a time of arrival of ultrasound at an ultrasound reception point.
Fig. 5 is a diagram showing relation between a position of an ultrasound transmission point and intensity of ultrasound at an ultrasound reception point.
Fig. 6 is a diagram illustrating a method of determining whether or not there is an internal defect in the weld.
Fig. 7 is a diagram showing a state of attachment like a film, of an impurity (slag) to a surface of the weld.
Fig. 8 is a flowchart (No. 1) showing an exemplary procedure of processing in a control device.
Fig. 9 is an overall configuration diagram (No. 2) of a welding system to which the welding inspection method is applied.
Fig. 10 is a flowchart (No. 2) showing an exemplary procedure of processing in the control device.

### DESCRIPTION OF THE PREFERRED EMBODIMENTS

An embodiment of the present disclosure will be described in detail below with reference to the drawings. The same or corresponding elements in the drawings have the same reference characters allotted and description thereof will not be repeated.

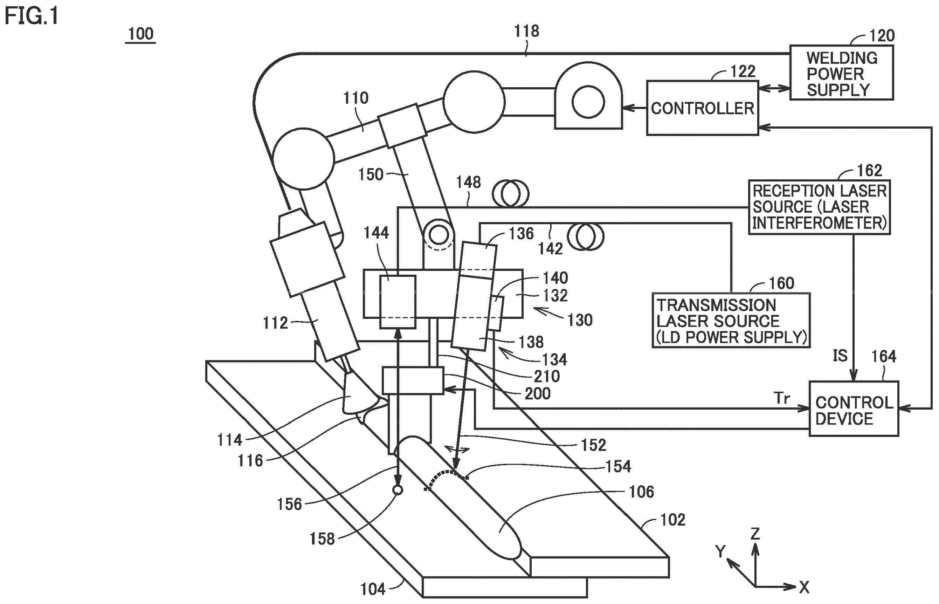

Fig. 1 is an overall configuration diagram of a welding system 100 to which a welding inspection method according to the present embodiment is applied. This welding system 100 is used, for example, for inspection of a weld (weld bead) 106 in lap fillet joint of thin plates (base materials 102 and 104). Base materials 102 and 104 are, for example, galvanized steel plates each having a thickness approximately from 1 to 2 mm. In the figure, a Y direction indicates a direction of movement of welding, a Z direction indicates a direction normal to base materials 102 and 104, and an X direction indicates a direction orthogonal to the Y direction and the Z direction.

Welding system 100 includes a robot manipulator 110, a welding torch 112, a wire 118, a welding power supply 120, and a controller 122.

Robot manipulator 110 is an articulated manipulator such as a six-axis articulated manipulator. Robot manipulator 110 is controlled by controller 122 so as to weld base materials 102 and 104 with welding torch 112 at a set welding speed.

Welding torch 112 supplies a welding wire and not-shown shielding gas (argon gas, carbon dioxide gas, or the like) toward a portion of joint between base materials 102 and 104. Welding torch 112 receives supply of a welding current from welding power supply 120 through wire 118, and generates an arc 114 between a tip end of the welding wire and the portion of joint between base materials 102 and 104.

Instead of the welding wire, a non-consumable electrode (tungsten or the like) may be used while a filler (filler metal) that forms weld metal is added. That is, arc welding by welding torch 112 may be a welding electrode type (such as MAG welding or MIG welding) using the welding wire, or may be a non-welding electrode type (such as TIG welding) with addition of the filler.

Welding power supply 120 generates a welding voltage and a welding current in order to perform arc welding, and outputs the generated welding voltage and welding current to welding torch 112.

Controller 122 includes a central processing unit (CPU), memories (a random access memory (RAM) and a read only memory (ROM)), and an input and output port for input and output of various signals (none of them are shown). The CPU develops a program stored in the ROM on the RAM and executes the same. Various types of processing executed by controller 122 are described in the program stored in the ROM.

Controller 122 controls the operation of robot manipulator 110 and output of welding power supply 120 such that base materials 102 and 104 are welded by welding torch 112 at a set welding speed. At this time, controller 122 controls robot manipulator 110 such that a position of a welding inspection head 130 (which will be described later) is in the rear of welding torch 112 in the direction of movement of welding.

In addition, controller 122, in coordination with a control device 164 that controls welding inspection head 130, exchanges various signals with control device 164. For example, controller 122 notifies control device 164 of start, stop, continuation, and a welding speed, of welding by welding torch 112. When controller 122 receives a defect detection signal indicating detection of an internal defect in the weld from control device 164, controller 122 can stop welding by welding torch 112 or change a welding condition.

Welding system 100 further includes welding inspection head 130, optical fibers 142 and 148, a link 150, a transmission laser source 160, a reception laser source 162, and control device 164.

Welding inspection head 130 includes a transmission laser light irradiation device 134 and a reception laser light probe 144. Transmission laser light irradiation device 134 and reception laser light probe 144 are attached to an attachment member 132. Attachment member 132 is coupled to robot manipulator 110 by link 150.

Transmission laser light irradiation device 134 includes microchip laser 136, a scanning mechanism 138, and a photodetector 140. Microchip laser 136 is, for example, small solid-state laser using a YAG crystal, receives excitation light from transmission laser source 160 through optical fiber 142, and generates high-output pulsed laser light.

Scanning mechanism 138 includes a mechanism that scans a position of irradiation with pulsed laser light generated by microchip laser 136 in two axes of a welding direction (Y direction) and a direction of width of welding (X direction). Scanning mechanism 138 includes, for example, a galvano mirror an angle of which is adjustable and a drive mechanism that drives the galvano mirror. During welding inspection, the drive mechanism that drives scanning mechanism 138 is controlled by control device 164 such that an ultrasound transmission point group 154 irradiated with transmission laser light 152 (pulsed laser light) includes the entire area in the width direction (X direction) of weld bead 106.

Photodetector 140 detects oscillation of transmission laser light 152 (pulsed laser light), and outputs a trigger signal Tr to control device 164 each time pulsed laser light is oscillated.

Reception laser light probe 144 irradiates an ultrasound reception point 158, which is a predetermined position in the X direction on base material 104, with reception laser light 156 (reference light) received from reception laser source 162 through optical fiber 148. In addition, reception laser light probe 144 receives reflected light reflected from base material 104, of reception laser light 156 emitted to base material 104, and outputs reflected light to reception laser source 162 through optical fiber 148.

Reception laser source 162 includes a laser interferometer, and outputs to reception laser light probe 144 through optical fiber 148, reception laser light 156 (reference light) to be emitted to base material 104. Then, reception laser source 162 receives from reception laser light probe 144, reflected light of reception laser light 156 from base material 104 received by reception laser light probe 144, and outputs to control device 164, a detection signal IS of coherent light including reference light and reflected light of reception laser light 156.

In consideration of in-process defect detection during welding, the positions of irradiation with transmission laser light 152 and reception laser light 156 are desirably close to a position of welding (a molten pool 116). For example, the positions of irradiation can be set to 20 to 30 mm behind molten pool 116 in the direction of movement of welding.

Similarly to controller 122, control device 164 includes a CPU, memories (a RAM and a ROM), and an input and output port for input and output of various signals (none of them are shown). The CPU develops a program stored in the ROM on the RAM and executes the same. Various types of processing executed by control device 164 are described in the program stored in the ROM.

Control device 164 measures detection signal IS of coherent light including reference light and reflected light of reception laser light 156 for a predetermined time period (for example, 10 µs), with trigger signal Tr from photodetector 140 indicating timing of oscillation of transmission laser light 152 (pulsed laser light) being defined as the reference. Control device 164 then measures intensity of ultrasound at ultrasound reception point 158 corresponding to trigger signal Tr, from measured detection signal IS of coherent light.

Control device 164 measures the intensity of ultrasound at ultrasound reception point 158 as described above while the position of irradiation (ultrasound transmission point) with transmission laser light 152 is scanned in the direction of width (X direction) of weld 106. When measurement corresponding to one scan ends, control device 164 determines whether or not there is an internal defect produced in weld 106 based on a degree of attenuation of ultrasound (reflected ultrasound) that is reflected by a lower surface of a lower plate (base material 104) and reaches ultrasound reception point 158.

In this welding system 100, a laser ultrasonic method is used to detect the internal defect in weld 106. Specifically, ultrasound is generated in the inside of a detection target by irradiation of weld 106 to be inspected with transmission laser light 152, and surface vibration in accordance with intensity of ultrasound at ultrasound reception point 158 irradiated with reception laser light 156 is detected based on coherent light including reference light and reflected light of reception laser light 156. Whether or not there is an internal defect in weld 106 is then determined based on a detection difference between a case where there is no internal defect in weld 106 and a case where there is an internal defect.

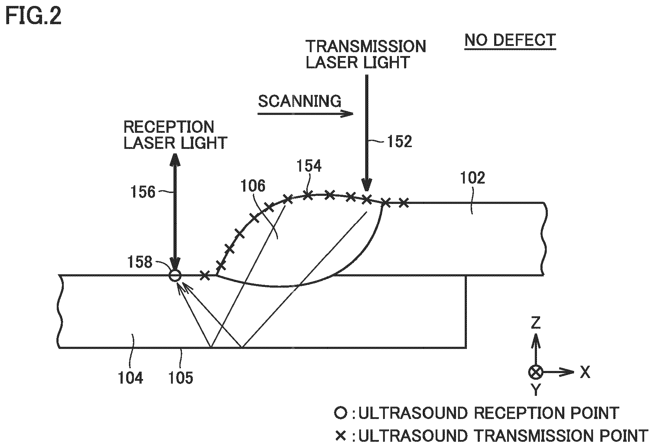

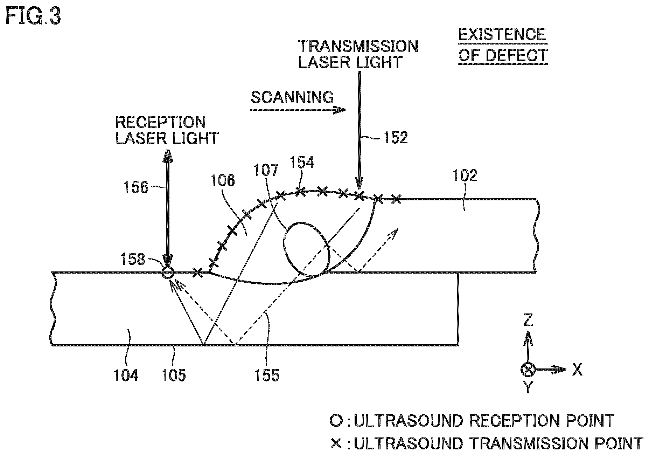

Figs. 2 and 3 are diagrams illustrating principles of detection of the internal defect in the weld by welding system 100. Fig. 2 illustrates a state where there is no defect in weld 106, and Fig. 3 illustrates a state where there is an internal defect (a blowhole or the like) in weld 106.

With reference to Fig. 2, in this welding system 100, an area including weld 106 is irradiated with transmission laser light 152 from above (Z direction), and ultrasound is excited at the position of irradiation with laser light. Generated ultrasound passes through weld 106, is reflected by a lower surface 105 of base material 104 on a lower side, and reaches an upper surface of base material 104. Intensity of ultrasound at the position of irradiation (ultrasound reception point 158) with reception laser light 156 is measured by measurement with reception laser light 156 (interference measurement using the laser interferometer), of micro vibration generated by this reached ultrasound at the surface of base material 104.

The position of irradiation with transmission laser light 152 is scanned in the X direction by scanning mechanism 138. An ultrasound transmission point group 154 indicated by a × mark indicates the position of irradiation with transmission laser light 152 which is pulsed laser light. After measurement at a certain position of irradiation ends, the next position of irradiation with transmission laser light 152 is scanned, and measurement at that position of irradiation is conducted.

The position of irradiation with reception laser light 156 is fixed in the X direction of base material 104. Ultrasound reception point 158 indicated by a ∘ mark indicates the position of irradiation with reception laser light 156. As a distance between each point in ultrasound transmission point group 154 and ultrasound reception point 158 is longer in the X direction, a time period of propagation of ultrasound from each point in ultrasound transmission point group 154 to ultrasound reception point 158 is longer, and intensity of ultrasound (micro vibration at the surface) at ultrasound reception point 158 also decreases due to diffusion attenuation.

With reference to Fig. 3, when there is a defect 107 such as a blowhole in weld 106, scattering attenuation in addition to diffusion attenuation also occurs at defect 107. Therefore, intensity of ultrasound 155 reaching ultrasound reception point 158 is lower (attenuation is larger) than that when there is no defect 107. Therefore, whether or not there is a defect 107 in weld 106 can be determined by capturing a degree of attenuation of ultrasound reaching ultrasound reception point 158.

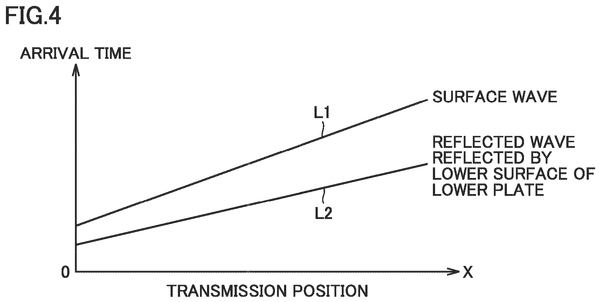

Fig. 4 is a diagram showing relation between the position of irradiation with transmission laser light 152 and time of arrival of ultrasound at ultrasound reception point 158. In Fig. 4, the horizontal axis represents the position of irradiation with transmission laser light 152 (the position of the ultrasound transmission point) in the X direction, and a larger value indicates a longer distance from ultrasound reception point 158. The vertical axis represents time from emission of transmission laser light 152 (generation of ultrasound at the ultrasound transmission point) to arrival of ultrasound at ultrasound reception point 158.

In Fig. 4, a line L1 indicates time of arrival of ultrasound (surface wave) transmitted through weld 106 over the surface of base material 104. A line L2 indicates time of arrival of ultrasound (reflected wave) that passes through weld 106 from the ultrasound transmission point and is reflected by lower surface 105 of base material 104.

Since time of arrival of surface wave is not affected by whether or not there is defect 107, distinction as to whether ultrasound detected at the ultrasound reception point is surface wave or ultrasound (reflected wave) reflected by lower surface 105 of base material 104 can be made based on relation between the position of the ultrasound transmission point and the time of arrival.

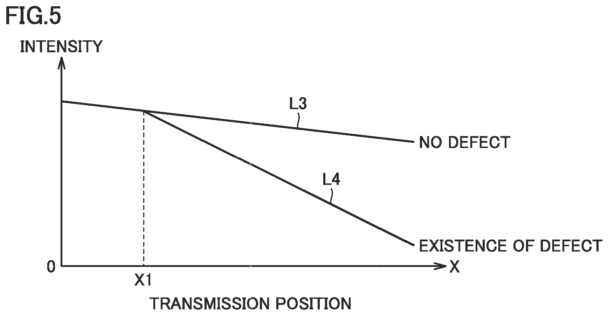

Fig. 5 is a diagram showing relation between the position of the ultrasound transmission point and intensity of ultrasound at the ultrasound reception point. In Fig. 5, similarly to Fig. 4, the horizontal axis represents the position of the ultrasound transmission point. The vertical axis indicates intensity of reflected waves (line L2) in Fig. 4 at the ultrasound reception point.

In Fig. 5, a line L3 indicates intensity of reflected waves (line L2) at the ultrasound reception point when there is no internal defect in weld 106. As a value of the position of the ultrasound transmission point is larger (as the distance from the ultrasound reception point is longer), signal intensity is attenuated by diffusion attenuation.

A line L4 indicates intensity of reflected waves (line L2 in Fig. 4) at the ultrasound reception point when there is an internal defect (a blowhole or the like) in weld 106. In a range where the position of the ultrasound transmission point is farther than a position X1 shown in Fig. 5, reflected waves reaching the ultrasound reception point pass through the internal defect produced in weld 106. At this time, scattering attenuation at the internal defect in addition to diffusion attenuation also occurs, and hence intensity of reflected waves (line L4) at the ultrasound reception point is lower than intensity of reflected waves (line L3) when there is no internal defect. That is, at the ultrasound reception point, the degree of attenuation of reflected waves when an internal defect is produced becomes higher than that when there is no internal defect.

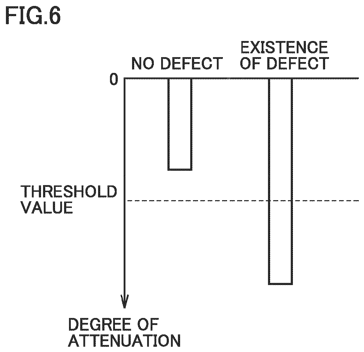

Fig. 6 is a diagram showing a method of determining whether or not there is an internal defect in weld 106. Whether or not there is an internal defect in weld 106 is determined based on magnitude of the degree of attenuation corresponding to the position of the ultrasound transmission point, with respect to intensity of reflected waves at the ultrasound reception point. That is, it is determined that there is no internal defect when the degree of attenuation in accordance with the position of the ultrasound transmission point is smaller than a predetermined threshold value, and it is determined that there is an internal defect produced in weld 106 to be inspected when the degree of attenuation is larger than the threshold value. The threshold value is set as appropriate to a value with which distinction as to whether or not there is an internal defect can be made, in an evaluation test or the like in advance.

### <Removal of Impurity at Surface of Weld>

An impurity such as slag produced during welding may be attached to the surface of weld 106. Slag refers to an oxide of silicon (Si), manganese (Mn), iron (Fe), or the like exposed at the surface of the weld.

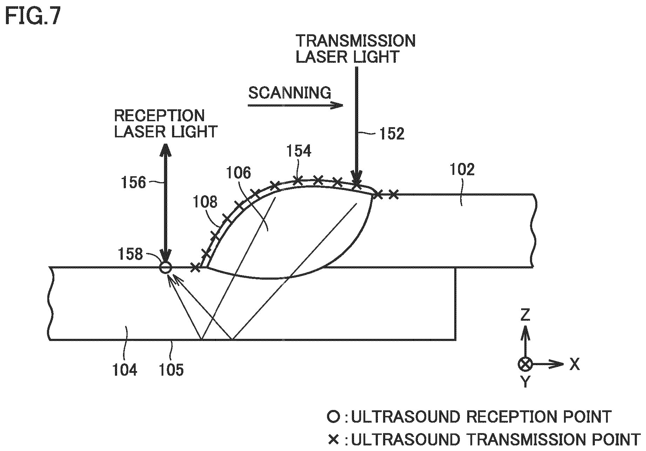

Fig. 7 is a diagram showing a state of attachment like a film, of an impurity (slag) 108 to the surface of weld 106. With impurity 108 being attached to the surface of weld 106, impurity 108 is irradiated with transmission laser light in welding inspection, which may interfere with generation of ultrasound. Consequently, there is a concern that the impurity becomes a factor for defective welding inspection.

Welding system 100 further includes a surface treatment apparatus 200 as shown in Fig. 1. Surface treatment apparatus 200 is a tool for removal of impurity 108 attached to the surface of weld 106. Surface treatment apparatus 200 is coupled to welding inspection head 130. In the example shown in Fig. 1, surface treatment apparatus 200 is coupled to welding inspection head 130 by a link 210.

Surface treatment apparatus 200 according to an aspect not covered by the present invention, includes a removal mechanism (a brush or the like) that comes in contact with the surface of weld 106 for mechanical removal at the surface of weld 106. Impurity 108 attached to the surface of weld 106 is removed by this removal mechanism. A method of removing the impurity is not limited to the method of mechanical removal at the surface of weld 106. According to the present invention, surface treatment apparatus 200 is implemented by a laser light irradiation device, output from which is adjusted to remove impurity 108 attached to the surface of weld 106. In this case, by irradiation of the surface of weld 106 with laser from surface treatment apparatus 200, impurity 108 attached to the surface of weld 106 can contactlessly be removed by impact produced by laser ablation.

In welding inspection, control device 164 performs processing in a first step to a fourth step below in this order.

(First Step) The impurity at the surface of weld 106 is removed with surface treatment apparatus 200.

(Second Step) The surface of weld 106 from which the impurity has been removed is irradiated with transmission laser light.

(Third Step) Ultrasound (reflected waves) reflected by lower surface 105 of base material 104 is detected.

(Fourth Step) Whether or not there is an internal defect in weld 106 is determined based on a result of detection of reflected waves.

As shown in Fig. 1, the position of surface treatment apparatus 200 according to the present embodiment is adjusted in advance such that the position of removal by surface treatment apparatus 200 is in the rear of molten pool 116 (the position of welding by welding torch 112) in the direction of movement of welding and in front of ultrasound transmission point group 154 (the position of irradiation with transmission laser light 152) in the direction of movement of welding. Thus, in in-process welding inspection during welding, the impurity at the surface of weld 106 can be removed by surface treatment apparatus 200 before irradiation of the surface of weld 106 with transmission laser light 152.

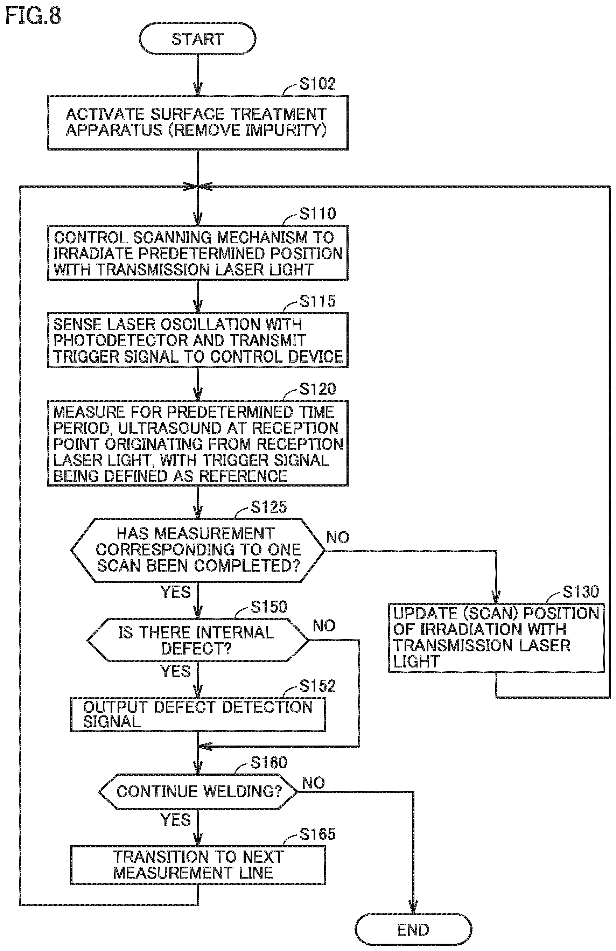

Fig. 8 is a flowchart showing an exemplary procedure of processing in in-process welding inspection during welding in control device 164 according to the present embodiment.

Control device 164 activates surface treatment apparatus 200 to have the impurity at the surface of weld 106 removed (step S102). During welding, welding inspection head 130 also moves together with welding torch 112 in the direction of movement of welding (Y direction). Surface treatment apparatus 200 is coupled to welding inspection head 130, and furthermore, the position of removal by surface treatment apparatus 200 is adjusted to the position in the rear of molten pool 116 in the direction of movement of welding and in front of ultrasound transmission point group 154 in the direction of movement of welding as described above. Therefore, at the position to be irradiated with transmission laser light 152 in a subsequent step, the impurity has already been removed. Processing in step S102 corresponds to the "first step" described above.

Control device 164 then controls scanning mechanism 138 so as to irradiate a predetermined position in the X direction of weld 106 with transmission laser light 152 (step S110). The position in the X direction of each point of irradiation with transmission laser light 152 in one scan is determined in advance, and a predetermined number of (for example, twenty) positions of irradiation are determined in an area including weld 106. Processing in step S110 corresponds to the "second step" described above.

Oscillation of (irradiation with) transmission laser light 152 (pulsed laser light) is then detected by photodetector 140, and trigger signal Tr indicating start of detection of ultrasound corresponding to this pulse irradiation is transmitted from photodetector 140 to control device 164 (step S115).

With trigger signal Tr being defined as the reference, control device 164 measures for a predetermined time period (for example, 10 µs), intensity of ultrasound at ultrasound reception point 158 based on coherent light of reception laser light 156 obtained by the laser interferometer (step S120). Processing in steps S115 and S120 corresponds to the "third step" described above.

Control device 164 then determines whether measurement corresponding to one scan with transmission laser light 152 has been completed (step S125). Specifically, whether measurement has been completed at all predetermined positions of irradiation in the X direction is determined. When measurement corresponding to one scan has not been completed (NO in step S125), control device 164 updates the position of irradiation with transmission laser light 152 to the next position (step S130). Then, the process returns to step S110.

When it is determined in step S125 that measurement corresponding to one scan with transmission laser light 152 has been completed (YES in step S125), control device 164 determines whether or not there is an internal defect in weld 106 corresponding to a range of one scan, based on a result of measurement corresponding to one scan (step S150).

Specifically, control device 164 extracts from the result of measurement corresponding to one scan, a signal of reflected waves (ultrasound that is reflected by the lower surface of base material 104 and reaches ultrasound reception point 158) reflected at the position of irradiation (ultrasound reception point) with reception laser light 156 for each position of irradiation (ultrasound transmission point) with transmission laser light 152 scanned in the X direction. Control device 164 then calculates the degree of attenuation of ultrasound in accordance with the position of irradiation with (position of transmission of) transmission laser light 152 for that extracted reflected wave signal, and determines whether or not the calculated degree of attenuation is larger than the threshold value. This threshold value is set as appropriate to a value that allows distinction as to whether or not there is an internal defect, in the evaluation test in advance. When the degree of attenuation is larger than the threshold value, control device 164 determines that there is an internal defect in weld bead 106 corresponding to the range of one scan. Processing in step S150 corresponds to the "fourth step" described above.

When it is determined in step S150 that there is an internal defect (YES in step S150), control device 164 outputs a defect detection signal indicating detection of a defect in weld 106 to controller 122 on a robot manipulator side (step S152). Thereafter, control device 164 has the process make transition to step S160.

When it is not determined in step S150 that there is an internal defect (NO in step S150), on the other hand, control device 164 has the process make transition to step S160 without performing processing in step S152.

Control device 164 then determines whether welding is to be continued, based on the signal from controller 122 indicating whether or not welding should continue (step S160). When welding is to be continued (YES in step S160), control device 164 makes transition to measurement in a next measurement line (step S165), and has the process return to step S110.

When it is determined in step S160 that welding is to be stopped based on the signal from controller 122 (NO in step S160), on the other hand, control device 164 quits the process of welding inspection.

As set forth above, the welding inspection method according to the present embodiment includes removing the impurity at the surface of weld 106 with surface treatment apparatus 200 (first step), irradiating the surface of weld 106 from which the impurity has been removed with transmission laser light (second step), detecting reflected ultrasound reflected by lower surface 105 of base material 104 (third step), and determining whether or not there is an internal defect in weld 106 based on a result of detection of reflected ultrasound (fourth step).

According to such a welding inspection method, even when the impurity produced during welding is attached to the surface of weld 106, the impurity is removed before irradiation of the surface of weld 106 with transmission laser light. Therefore, even when the impurity produced during welding is attached to the surface of weld 106, the internal defect in weld 106 can appropriately be detected.

### [First Modification]

In the embodiment described above, an example where dedicated surface treatment apparatus 200 for removal of impurity 108 attached to the surface of weld 106 is provided is described. Without dedicated surface treatment apparatus 200, however, impurity 108 attached to the surface of weld 106 may be removed with transmission laser light.

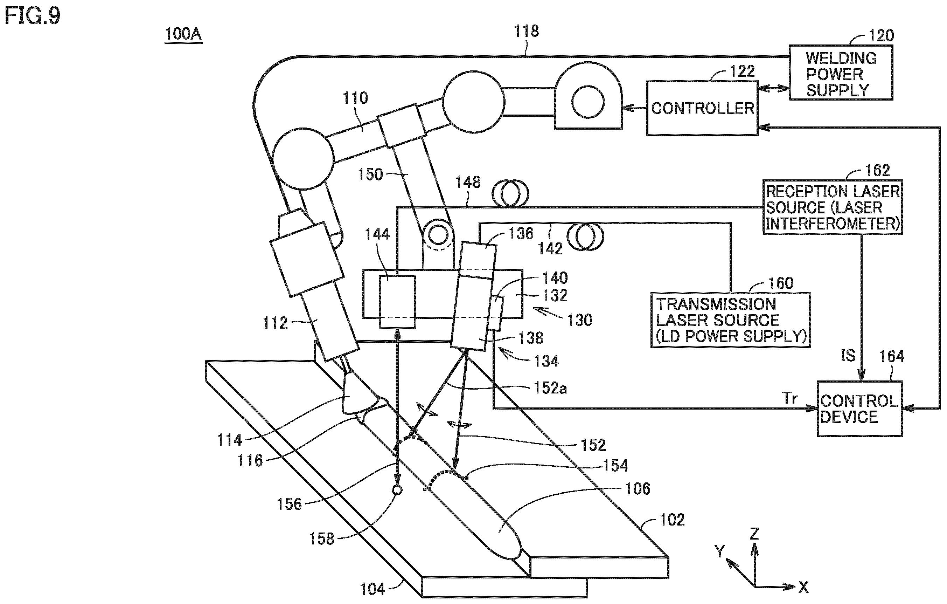

Fig. 9 is an overall configuration diagram of a welding system 100A to which the welding inspection method according to the present second modification is applied. This welding system 100A is configured by removal of surface treatment apparatus 200 and link 210 from welding system 100 described above.

In this welding system 100A, in view of provision of scanning mechanism 138 (the galvano mirror or the like) for scanning with transmission laser light in transmission laser light irradiation device 134, scanning mechanism 138 is controlled to remove the impurity attached to the surface of weld 106 by irradiation of the surface of weld 106 with transmission laser light 152a, and controlled to irradiate the surface of weld 106 from which the impurity has been removed with transmission laser light 152. Transmission laser light can thus be used for removal of the impurity. Consequently, without dedicated surface treatment apparatus 200, the internal defect in weld 106 can appropriately be detected.

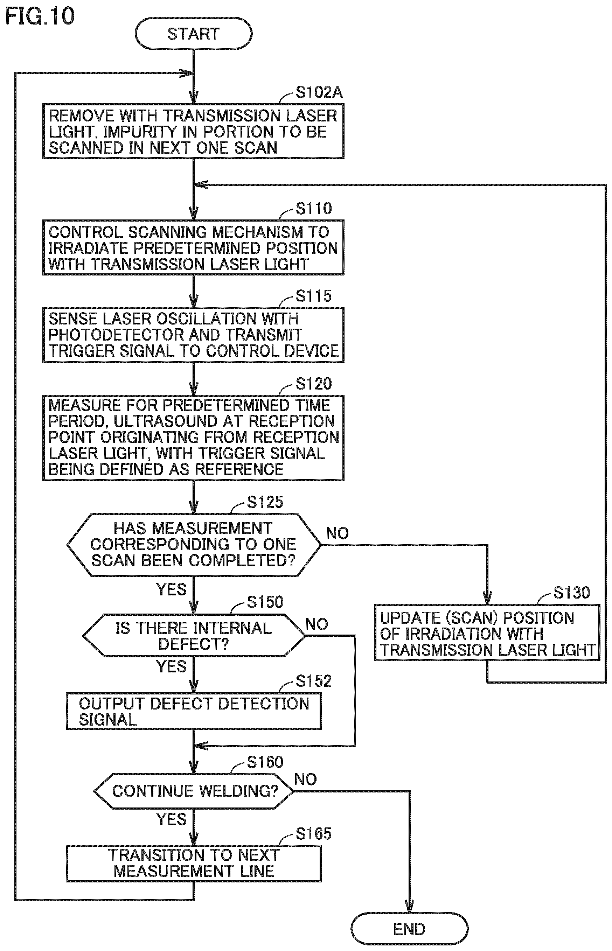

Fig. 10 is a flowchart showing an exemplary procedure of processing in in-process welding inspection during welding by control device 164 according to the present first modification. The flowchart in Fig. 10 includes step S102A in place of step S102 in Fig. 8 described above.

Initially, control device 164 controls scanning mechanism 138 to remove the impurity attached to the surface to be scanned in next one scan, by irradiation with transmission laser light 152a, of the surface of weld 106 to be scanned in next one scan (step S102A).

Control device 164 then conducts welding inspection by irradiation of the surface corresponding to one scan with transmission laser light 152 (steps S110 to S152).

When welding is to be continued (YES in step S160), control device 164 makes transition to measurement in the next measurement line (step S165) and has the process return to step S102A. When it is determined to stop welding (NO in step S160), control device 164 quits the process of welding inspection.

As set forth above, impurity 108 attached to the surface of weld 106 may be removed by irradiation of the surface of weld 106 with transmission laser light 152.

### [Second Modification]

In the welding inspection method according to the embodiment described above, surface treatment apparatus 200 constantly removes the impurity without verification as to whether or not impurity 108 is attached to the surface of weld 106.

In contrast, surface treatment apparatus 200 may remove the impurity only when whether or not impurity 108 is attached to the surface of weld 106 is verified in advance and then a result of verification indicating attachment of impurity 108 is obtained. Specifically, for example, initially, first welding inspection is conducted without removal of the impurity by surface treatment apparatus 200, and whether or not the impurity is attached to the surface of weld 106 is estimated based on the result of the first welding inspection. When attachment of no impurity is estimated based on the result of the first welding inspection, the welding inspection ends. When attachment of the impurity is estimated based on the result of the first welding inspection, on the other hand, surface treatment apparatus 200 removes the impurity and second welding inspection is conducted. According to such a modification, removal of the impurity by surface treatment apparatus 200 even in the case of attachment of no impurity 108 to the surface of weld 106 can more readily be avoided. Consequently, the welding inspection can efficiently be conducted.

### [Third Modification]

Though ultrasound reception point 158 is irradiated with reception laser light from reception laser light probe 144 and reflected waves are contactlessly detected in the welding inspection method according to the embodiment described above, the method of detecting reflected waves is not necessarily limited to a contactless method. For example, a device (a contactor) that is in contact with ultrasound reception point 158 to detect reflected waves at ultrasound reception point 158 may be arranged to detect reflected waves.

### [Fourth Modification]

Though ultrasound (reflected waves) excited by transmission laser light and reflected by lower surface 105 of base material 104 is detected in the welding inspection method according to the embodiment described above, ultrasound to be detected should only be ultrasound excited by transmission laser light, and it is not necessarily limited to ultrasound reflected by lower surface 105 of base material 104.

### [Fifth Modification]

Though surface treatment apparatus 200 is coupled to welding inspection head 130 in welding system 100 according to the embodiment described above, surface treatment apparatus 200 is not necessarily limited to the surface treatment apparatus coupled to welding inspection head 130. In other words, welding inspection head 130 and surface treatment apparatus 200 may be mounted on robots different from each other.

The embodiment described above and forms shown in the first to fifth modifications thereof can be combined as appropriate within the technically consistent scope.

Though an embodiment of the present invention has been described, it should be understood that the embodiment disclosed herein is illustrative and non-restrictive in every respect. The scope of the present invention is defined by the terms of the appended claims.

## Claims

1. A welding inspection method of inspecting a weld (106), the welding inspection method comprising:
removing an impurity (108) at a surface of the weld (106);
irradiating the surface of the weld (106) from which the impurity (108) has been removed with transmission laser light (152, 152a) for excitation of ultrasound in inside of a detection target;
detecting ultrasound excited by transmission laser light (152, 152a); and
determining whether there is an internal defect (107) in the weld (106) based on a result of detection of ultrasound, and
wherein the removing an impurity (108) includes removing the impurity (108) by irradiating the surface of the weld (106) with transmission laser light (152, 152a).

2. The welding inspection method according to claim 1, wherein
the detecting ultrasound includes irradiating a predetermined position (158) on the detection target where reflected ultrasound reflected by a lower surface (5) of a base material of the detection target can be detected, with reception laser light (156) for detection of reflected ultrasound.

3. The welding inspection method according to claim 1, performed in-process while welding is being performed.

## Patentansprüche

1. Schweißprüfverfahren zum Prüfen einer Schweißnaht (106), wobei das Schweißprüfverfahren umfasst:
Entfernen einer Verunreinigung (108) auf einer Oberfläche der Schweißnaht (106);
Bestrahlen der Oberfläche der Schweißnaht (106), von der die Verunreinigung (108) entfernt wurde, mit Laserdurchstrahllicht (152, 152a) für eine Ultraschallanregung im Inneren eines Nachweisziels;
Nachweisen eines Ultraschalls, der durch Laserdurchstrahllicht (152, 152a) angeregt wird; und
Bestimmen, ob ein interner Defekt (107) in der Schweißnaht (106) vorliegt, auf der Grundlage eines Ultraschallnachweisergebnisses, und
wobei das Entfernen einer Verunreinigung (108) das Entfernen der Verunreinigung (108) durch Bestrahlen der Oberfläche der Schweißnaht (106) mit Laserdurchstrahllicht (152, 152a) beinhaltet.

2. Schweißprüfverfahren nach Anspruch 1, wobei
das Nachweisen von Ultraschall das Bestrahlen einer vorbestimmten Position (158) auf dem Nachweisziel, an der reflektierter Ultraschall, der von einer Unterseite (5) eines Basismaterials des Nachweisziels reflektiert wird, nachgewiesen werden kann, mit Laseraufnahmelicht (156) zum Nachweis von reflektiertem Ultraschall beinhaltet.

3. Schweißprüfverfahren nach Anspruch 1, das während eines Schweißvorgangs durchgeführt wird.

## Revendications

1. Procédé d'inspection de soudure pour inspecter une soudure (106), le procédé d'inspection de soudure comprenant les étapes consistant à :
retirer une impureté (108) au niveau d'une surface de la soudure (106) ;
irradier la surface de la soudure (106) à partir de laquelle l'impureté (108) a été retirée avec une lumière laser de transmission (152, 152a) pour l'excitation d'ultrasons à l'intérieur d'une cible de détection ;
détecter des ultrasons excités par la lumière laser de transmission (152, 152a) ; et
déterminer s'il existe un défaut interne (107) dans la soudure (106) sur la base d'un résultat de détection d'ultrasons, et
dans lequel le retrait d'une impureté (108) inclut le retrait de l'impureté (108) en irradiant la surface de la soudure (106) avec une lumière laser de transmission (152, 152a).

2. Procédé d'inspection de soudure selon la revendication 1, dans lequel
la détection d'ultrasons inclut l'irradiation d'une position prédéterminée (158) sur la cible de détection où des ultrasons réfléchis, réfléchis par une surface inférieure (5) d'un matériau de base de la cible de détection, peuvent être détectés, avec une lumière laser de réception (156) pour la détection d'ultrasons réfléchis.

3. Procédé d'inspection de soudure selon la revendication 1, réalisé en cours de processus pendant qu'un soudage est en train d'être réalisé.
